# EUROPEAN PATENT APPLICATION

(11) **EP 4 850 675 A1**
(43) Date of publication of application: **09.12.2026**
(21) Application number: 25748259.6
(22) Date of filing: 22.01.2025
(51) Int. Cl.: A61K 35/28, A61L 26/00, A61P 9/00, A61P 17/02, A61P 29/00, C12N 5/077, C12N 5/0775

(54) **MEDICINAL COMPOSITION AND TREATMENT METHOD USING SAME**

(30) Priority: 30.01.2024 JP 2024012210; 24.06.2024 JP 2024101298
(71) Applicant: Asfreya Inc., Tokyo 136-0082 (JP)
(72) Inventor: OCHIYA Takahiro, Tokyo 136-0082 (JP)
(74) Representative: J A Kemp LLP
(86) International application number: PCT/JP2025/001814
(87) International publication number: WO 2025/164451

(57) **Abstract**

An object of the present invention is to provide a novel drug which can be used for various treatments. The medicinal composition according to the present invention is characterized by comprising extracellular vesicles derived from equine mesenchymal stem cells. The medicinal composition according to the present invention can be a tissue repair agent, an anti-inflammatory agent, an angiogenic agent, or a wound healing agent.

## Description

### [Technical Field]

The present invention relates to a medicinal composition and a treatment method using the medicinal composition.

### [Background Art]

There is a growing need to developing a new drug for treating a living organism, such as treating tissue repair, anti-inflammation, angiogenesis, and wound healing.

### [SUMMARY OF THE INVENTION]

### [Technical Problem]

An object of the present invention is to provide a novel drug which can be used for various treatments.

### [Solution to Problem]

In order to achieve the above object, a medicinal composition according to the present invention is characterized by comprising extracellular vesicles derived from equine mesenchymal stem cells.

The method of treating a living organism according to the present invention is characterized by comprising an administration step of administering the medicinal composition according to the present invention to the living organism.

### [Advantageous Effects of Invention]

The medicinal composition according to the present invention can, for example, heal wounds, relieve inflammation, and promote angiogenesis in a living organism.

### [BRIEF DESCRIPTION OF DRAWINGS]

[FIG. 1] FIG. 1 shows a graph illustrating the particle size distribution of a sample of extracellular vesicles derived from equine mesenchymal stem cells from Example 1.
[FIG. 2] FIG. 2 shows a graph illustrating results from detection of a C9 marker in the sample of extracellular vesicles derived from equine mesenchymal stem cells from Example 1.
[FIG. 3] FIG. 3 shows a graph illustrating the wound healing effects of a sample of extracellular vesicles derived from equine mesenchymal stem cells from Example 2.
[FIG. 4] FIG. 4 shows a graph illustrating the concentrations of cytokines in a culture supernatant from Example 3.
[FIG. 5] FIG. 5 shows the phase-contrast micrographs of HUVEC tubes from Example 4.
[FIG. 6] FIG. 6 shows a graph illustrating the relative values of the lengths of the HUVEC tubes from Example 4.
[FIG. 7] FIG. 7 shows a graph illustrating the number of branching points of the HUVEC tubes from Example 4.
[FIG. 8A] FIG. 8A shows a graph illustrating the cell viability of dermal fibroblasts in the presence of a sample of extracellular vesicles derived from equine mesenchymal stem cells from Example 5.
[FIG. 8B] FIG. 8B shows a graph illustrating the cell viability of IHFPDCs (dermal papilla cells) in the presence of the sample of extracellular vesicles derived from equine mesenchymal stem cells from Example 5.
[FIG. 8C] FIG. 8C shows a graph illustrating the cell viability of NHEKs (skin-derived keratinocytes) in the presence of the sample of extracellular vesicles derived from equine mesenchymal stem cells from Example 5.

### [DESCRIPTION OF EMBODIMENTS]

The present invention includes, for example, the following embodiments.
[1] A medicinal composition characterized by comprising extracellular vesicles derived from equine mesenchymal stem cells.
[2] The medicinal composition according to [1], wherein the medicinal composition is a tissue repair agent.
[3] The medicinal composition according to [1] or [2], wherein the medicinal composition is an anti-inflammatory agent.
[4] The medicinal composition according to any one of [1] to [3], wherein the medicinal composition is an angiogenic agent.
[5] The medicinal composition according to any one of [1] to [4], wherein the medicinal composition is a wound healing agent.
[6] A method of treating a living organism, characterized by comprising an administration step of administering the drug composition according to any one of [1] to [5] to the living organism.
[7] The method of treating a living organism according to [6], wherein the administration step comprises administering the medicinal composition to the living organism as a tissue repair agent.
[8] The method of treating a living organism according to [6], wherein the administration step comprises administering the drug composition to the living organism as an anti-inflammatory agent.
[9] The method of treating a living organism according to [6], wherein the administration step comprises administering the drug composition to the living organism as an angiogenic agent.
[10] The method of treating a living organism according to any one of [6] to [9], wherein the administration step comprises administering the drug composition to the living organism as a wound healing agent.
[11] The method of treating a living organism according to any one of [6] to [10], wherein the living organism is a human or a non-human animal.
[12] The method of treating a living organism according to [11], wherein the non-human animal is a horse.

Unless otherwise specified, the terms used in the present specification can be used in the meanings commonly used in the art.

As used in the present specification, the term "treatment" is used, for example, in the broad sense, and include the meaning of prevention in addition to treatment in the narrow sense. Treatment in the narrow sense includes, the meanings of, for example, the healing (or complete recovery) of a target disease, the alleviation or improvement of a disease, the suppression of disease progression (prevention of worsening), or the like whereas disease prevention includes the meanings of, for example, the prevention of disease incidence, the prevention of disease onset, the prevention of disease recurrence, and the like. The treatment or prevention of diseases can also be described as the treatment or prevention of the symptoms of diseases, for example.

The present invention will be described below with reference to specific examples, but shall not be limited to these examples. Further, those examples of the present invention may be applied to one another.

### (1) Medicinal composition and method of treating living organism

As described above, the medicinal composition (pharmaceutical composition) according to the present invention is characterized by comprising extracellular vesicles derived from equine mesenchymal stem cells. Hereinafter, mesenchymal stem cells may also be referred to as MSCs.

Extracellular vesicles are, for example, vesicles secreted by cells, and are membrane vesicles enclosed by lipid bilayers. Extracellular vesicles include, for example, exosomes, microvesicles, apoptotic vesicles, and the like.

Mesenchymal stem cells, which are, for example, somatic stem cells with multipotent differentiation potential, are capable of differentiating into cells of mesenchymal tissues (mesodermal tissues). There is no particular limitation for tissues from which mesenchymal stem cells are to be derived, but examples include bone marrow, adipose tissues, placental tissues, umbilical cord tissues, dental pulp, and the like.

Extracellular vesicles derived from equine mesenchymal stem cells can be prepared, for example, by culturing equine mesenchymal stem cells in a medium, collecting the supernatant including extracellular vesicles, and further isolating a fraction of the extracellular vesicles from the supernatant.

Preferably, the medicinal composition according to the present invention comprises, for example, a fraction of the extracellular vesicles isolated from equine mesenchymal stem cells, but does not substantially comprise equine mesenchymal stem cells. The phrase "do not substantially comprise equine mesenchymal stem cells" also implies, for example, that no cell proliferation is observed even when the medicinal composition is cultured.

As for equine mesenchymal stem cells, mesenchymal stem cells isolated from horse or a cell line may be used.

There is no particular limitation for the medium for equine mesenchymal stem cells, and any media for use in culture of stem cells may be used, and preferably any media for use in culture of mesenchymal stem cells may be used. Specific examples of the media include, for example, a basal media such as DMEM, RPMI-1640, and the like, and commercially available products may be used.

The basal media may contain, for example, serum, plasma, or artificial serum. Examples of serum and plasma include those derived from a human or a non-human animal. Examples of the non-human animal include cattle, horse, or the like. The basal media may also be free of, for example, serum, plasma, and artificial serum.

There is no particular limitation for a culture temperature, but it may be, for example, 30 to 40°C (37°C as a specific example). There is no particular limitation for days of culture. Passage culture, for example, may preferably be performed when culturing. There is no particular limitation for the frequency of passage, but it may be, for example, every four to five days.

There is no particular limitation for a method of isolating the extracellular vesicle fraction from the supernatant, but examples include separation methods such as, for example, ultrafiltration methods, ultracentrifugation methods, concentration gradient methods, and microfluidic systems.

The extracellular vesicle fraction includes a plurality of extracellular vesicles. There is no particular limitation for the size of the extracellular vesicles in the extracellular vesicle fraction, but their particle sizes may include, for example, 30 to 250 nm, 50 to 200 nm, and 100 to 150 nm. There is no particular limitation for the peak of particle sizes in the particle size distribution of the extracellular vesicle fraction, but it may be, for example, 70 to 150 nm, 95 to 135 nm, or 100 to 115 nm. Further, there is no particular limitation for the proportion of vesicles having the peak (e.g., 100 to 115 nm) in the particle size distribution when the total number of vesicles is considered as 100%, but the lower limit thereof may be, for example, 30% or more, 40% or more, 60% or more. The extracellular vesicles used as an active ingredient in a wound healing agent according to the present invention are preferably included in a fraction obtained from the supernatant and fractionated so as to have the particle size and the particle size distribution as described above.

There is no particular limitation for a method of measuring particle sizes of extracellular vesicles, but this can be performed by, for example, light scattering methods, measuring methods based on Brownian motion, electrical resistance methods, and the like. Measurement methods based on Brownian motion include, for example, nanoparticle tracking analysis, and the like, for which commercially available nanoparticle analysis instruments (such as Product name: NanoSight, Malvern) can be used. When NanoSight is used, the measurement conditions may include, for example, the following:
Measurement time: 60 seconds
Number of repetitions: 3 times
Detection threshold: 5
Camera type: sCMOS
Laser type: Blue405
Camera level: 13
Syringe pump speed: 40

The medicinal composition according to the present invention can be used for administration to a living organism, for example, as a drug for living organisms. The living organism is, for example, a human or a non-human animal. Examples of the non-human animal include mammals such as horse, mouse, rat, dog, cat, monkey, rabbit, cattle, goat, and camel.

There is no particular limitation for the dosage of the medicinal composition according to the present invention, but the medicinal composition is preferably administered in a pharmaceutically effective amount. The pharmaceutically effective amount can be determined, for example, based on factors such as a target site for treatment and the severity of symptoms. There is no particular limitation for the content of the extracellular vesicles derived from equine mesenchymal stem cells in the medicinal composition according to the present invention, but the content is preferably, for example, such that a pharmaceutically effective amount can be administered.

There is no particular limitation for the administration mode of the medicinal composition according to the present invention, but it may be, for example, oral or parenteral. Examples of parenteral administration include topical, transdermal, subcutaneous, intravenous, intraarterial, intraperitoneal, intestinal, intranasal, and the like. The administration mode can be appropriately selected based on, for example, factors such as target diseases, symptoms and affected sites thereof.

The medicinal composition according to the present invention may include, for example, only extracellular vesicles derived from equine mesenchymal stem cells as an active ingredient, or may further include other ingredients. Further, the wound healing agent according to the present invention may include, for example, only the aforementioned active ingredient, or may include the aforementioned active ingredient and other additives. Examples of the additives include, pharmaceutically acceptable ingredients and the like. There is no particular limitation for specific examples of the additives, but they include, for example, excipients, carriers (substrates), and the like. Examples of the excipients and the carriers include aqueous solvents such as water, physiological saline, and buffers; oils and fats such as soybean oil; vaseline; alcohols such as glycerol; sugars such as maltose, dextrose, and dextrin; sugar alcohols such as xylitol; phospholipids; liposomes; and the like. In addition to the above, examples of the additives include binders, disintegrants, surfactants, emulsifiers, antioxidants, lubricants, wetting agents, thickening agents, stabilizers, UV blockers, antiseptic agents, preservatives, vitamins, minerals, colorants, and the like. The additives may be selected as appropriate, for example, depending on the administration mode of the medicinal composition according to the present invention.

There is no particular limitation for the dosage form of the medicinal composition according the present invention, but it may be selected as appropriate, for example, depending on the administration mode. The dosage forms of the medicinal composition according to the present invention include, for example, liquid, emulsion, gel, sol, ointment, solid dosage forms, and the like. Examples of the solid dosage forms include pill, tablet, powder, and the like.

The method of treating a living organism according to the present invention is characterized by comprising an administration step of administering the medicinal composition according to the present invention to the living organism, as described above. The method of treating a living organism according to the present invention is characterized by the use of the medicinal composition according to the present invention. There is no particular limitation for other steps and conditions. The description about the medicinal composition according to the present invention can be applied to the method of treating a living organism according to the present invention.

The medicinal composition according to the present invention has functions of tissue repair, angiogenesis, and anti-inflammation. Therefore, the medicinal composition according to the present invention can be used for these applications. Further, the medicinal composition according to the present invention, which has these functions, can be used for wound healing, for example, via three approaches: tissue repair, angiogenesis, and anti-inflammation. Wound healing (also referred to as complete recovery) generally occurs through a series of stages: the coagulation stage, the inflammatory stage, the proliferative stage, and the maturation stage. As described above, the medicinal composition according to the present invention, which has functions such as tissue repair, angiogenesis, and anti-inflammation, can be used for wound healing. Specifically, it can, for example, serve to promote wound healing.

As used in the present specification, the term "tissue repair" refers to, for example, restoring both the function and structure of lost tissues in a living organ to their original state. Further, as used in the present specification, the term "wound" refers to, for example, physical damage, and there is no particular limitation for a target site of a wound. The term "wound healing" may be, for example, the healing of wounds in body surface tissues (for example, skin), the healing of wounds in muscles or organs, or the healing of damage extending from skin to muscles or organs.

Specific examples of the medicinal composition according to the present invention are provided in (2) to (5) below, each of which may be applied to each other.

### (2) Tissue repair agent and method of repairing tissue

The medicinal composition according to the present invention can be used, for example, as a tissue repair agent, and may also be referred to as a tissue repair agent according to the present invention.

There is no particular limitation for the dosage of the tissue repair agent according to the present invention, but the tissue repair agent is preferably administered in a pharmaceutically effective amount. The pharmaceutically effective amount can be determined, for example, based on factors such as a target tissue site for repair, and the severity of symptoms. There is no particular limitation for the content of the extracellular vesicles derived from equine mesenchymal stem cells in the tissue repair agent according to the present invention, but the content is preferably, for example, such that a pharmaceutically effective amount can be administered.

There is no particular limitation for the administration mode of the tissue repair agent according to the present invention, but it may be, for example, oral or parenteral, and is preferably parenteral. A specific example is application to tissues to be repaired.

Specific examples of conditions when applying the tissue repair agent according to the present invention to an affected area (e.g., body surface such as skin) include, for example, the following:
The total daily amount of the extracellular vesicles: 1e+9 to 1e+10 particles
The application amount of the extracellular vesicles per unit area per application: 1e+8 to 1e+9 particles/cm²
   The number of applications per day: 1 to 3
   The duration of application: until tissue repair is complete.
   The interval of applications: application daily, or every 2 to 3 days

The tissue repair agent according to the present invention may include, for example, only extracellular vesicles derived from equine mesenchymal stem cells as an active ingredient, or may further include other ingredients, as described above. Further, the tissue repair agent according to the present invention may include, for example, only the aforementioned active ingredient, or may include the aforementioned active ingredient and other additives, as described above. Examples of the additives include, pharmaceutically acceptable ingredients and the like. There is no particular limitation for specific examples of the additives, but they include, for example, excipients, carriers (substrates), and the like. Examples of the excipients and the carriers include aqueous solvents such as water, physiological saline, and buffers; oils and fats such as soybean oil; vaseline; alcohols such as glycerol; sugars such as maltose, dextrose, and dextrin; sugar alcohols such as xylitol; phospholipids; liposomes; and the like. In addition to the above, examples of the additives include binders, disintegrants, surfactants, emulsifiers, antioxidants, lubricants, wetting agents, thickening agents, stabilizers, UV blockers, antiseptic agents, preservatives, vitamins, minerals, colorants, and the like. The additives may be selected as appropriate, for example, depending on the administration mode of the tissue repair agent according to the present invention.

There is no particular limitation for the dosage form of the tissue repair agent according the present invention, but it may be selected as appropriate, for example, depending on the administration mode. Examples of the tissue repair agent according to the present invention include topical agents (topical drugs). In view of application, the dosage forms of the tissue repair agent according to the present invention may include, for example, liquid, emulsion, gel, sol, ointment, and the like.

The method of treating a living organism according to the present invention may be described as, for example, a method of repairing a tissue. In this case, the method of repairing a tissue according to the present invention comprises an administration step of administering the medicinal composition according to the present invention (i.e., the tissue repair agent as described above) to a living organism. The method of repairing a tissue according to the present invention is characterized by the use of the medicinal composition according to the present invention. There is no particular limitation for other steps and conditions. The administration step may be, for example, oral administration or parenteral administration. A specific example is application to an affected area. The description about the tissue repair agent according to the present invention can be applied to the method of repairing a tissue according to the present invention.

### (3) Anti-inflammatory agent and method of treating inflammation

The medicinal composition according to the present invention can be used, for example, as an anti-inflammatory agent, and may also be referred to as an anti-inflammatory agent according to the present invention.

There is no particular limitation for the dosage of the anti-inflammatory agent according to the present invention, but the anti-inflammatory agent is preferably administered in a pharmaceutically effective amount. The pharmaceutically effective amount can be determined, for example, based on factors such as the sites of inflammation and the severity of inflammation. There is no particular limitation for the content of the extracellular vesicles derived from equine mesenchymal stem cells in the anti-inflammatory agent according to the present invention, but the content is preferably, for example, such that a pharmaceutically effective amount can be administered.

There is no particular limitation for the administration mode of the anti-inflammatory agent according to the present invention, but it may be, for example, oral or parenteral.

Specific examples of conditions when applying the anti-inflammatory agent according to the present invention to an affected area (e.g., body surface such as skin) include, for example, the following:
The total daily amount of the extracellular vesicles: 1e+9 to 1e+10 particles
The application amount of the extracellular vesicles per unit area per application: 1e+8 to 1e+9 particles/cm²
The number of applications per day: 1 to 3
The duration of application: until inflammation is relieved
The interval of applications: application daily, or every 2 to 3 days

Specific examples of conditions when the anti-inflammatory agent according to the present invention is administered orally include, for example, the following:
The total daily amount of the extracellular vesicles: 1e+9 to 1e+10 particles
The number of administrations per day: 1 to 3
The duration of administration: until inflammation is relieved
The interval of administration: administration daily, or every 3 to 7 days

The anti-inflammatory agent according to the present invention may include, for example, only extracellular vesicles derived from equine mesenchymal stem cells as an active ingredient, or may further include other ingredients, as described above. Further, the anti-inflammatory agent according to the present invention may include, for example, only the aforementioned active ingredient, or may include the aforementioned active ingredient and other additives, as described above. Examples of the additives include, pharmaceutically acceptable ingredients and the like. There is no particular limitation for specific examples of the additives, but they include, for example, excipients, carriers (substrates), and the like. Examples of the excipients and the carriers include aqueous solvents such as water, physiological saline, and buffers; oils and fats such as soybean oil; vaseline; alcohols such as glycerol; sugars such as maltose, dextrose, and dextrin; sugar alcohols such as xylitol; phospholipids; liposomes; and the like. In addition to the above, examples of the additives include binders, disintegrants, surfactants, emulsifiers, antioxidants, lubricants, wetting agents, thickening agents, stabilizers, UV blockers, antiseptic agents, preservatives, vitamins, minerals, colorants, and the like. The additives may be selected as appropriate, for example, depending on the administration mode of the anti-inflammatory agent according to the present invention.

There is no particular limitation for the dosage form of the anti-inflammatory agent according the present invention, but it may be selected as appropriate, for example, depending on the administration mode. There is no particular limitation for the dosage form, but examples include those exemplified for the medicinal composition according to the present invention. They specifically include, for example, liquid, emulsion, gel, sol, ointment, solid dosage form, and the like.

The method of treating a living organism according to the present invention may be described as, for example, a method of treating inflammation. In this case, the method of treating inflammation according to the present invention comprises an administration step of administering the medicinal composition according to the present invention (i.e., the anti-inflammatory agent as described above) to a living organism. The method of treating a wound according to the present invention is characterized by the use of the medicinal composition according to the present invention. There is no particular limitation for other steps and conditions. The administration step may be, for example, oral administration or parenteral administration. A specific example is application to an affected area. The description about the anti-inflammatory agent according to the present invention can be applied to the method of treating inflammation according to the present invention.

### (4) Angiogenic agent and angiogenesis method

The medicinal composition according to the present invention can be used, for example, as an angiogenic agent, and may also be referred to as an angiogenic agent according to the present invention.

There is no particular limitation for the dosage of the angiogenic agent according to the present invention, but the angiogenic agent is preferably administered in a pharmaceutically effective amount. The pharmaceutically effective amount can be determined, for example, based on factors such as a site where angiogenesis is required and the extent to which angiogenesis is needed. There is no particular limitation for the content of the extracellular vesicles derived from equine mesenchymal stem cells in the angiogenic agent according to the present invention, but the content is preferably, for example, such that a pharmaceutically effective amount can be administered.

There is no particular limitation for the administration mode of the angiogenic agent according to the present invention, but it may be, for example, oral or parenteral.

Specific examples of conditions when applying the angiogenic agent according to the present invention to an affected area (e.g., body surface such as skin) include, for example, the following:
The total daily amount of the extracellular vesicles: 1e+9 to 1e+10 particles
The application amount of the extracellular vesicles per unit area per application: 1e+8 to 1e+9 particles/cm²
The number of applications per day: 1 to 3
The duration of application: 1 to 30 days
The interval of applications: application daily, or every 2 to 3 days

Specific examples of conditions when the angiogenic agent according to the present invention is administered to an affected area by injection include, for example, the following:
The total daily amount of the extracellular vesicles: 1e+9 to 1e+10 particles
The number of administrations per day: 1 to 3
The duration of administration: 1 to 30 days
The interval of administration: administration daily, or every 3 to 7 days

The angiogenic agent according to the present invention may include, for example, only extracellular vesicles derived from equine mesenchymal stem cells as an active ingredient, or may further include other ingredients, as described above. Further, the angiogenic agent according to the present invention may include, for example, only the aforementioned active ingredient, or may include the aforementioned active ingredient and other additives, as described above. Examples of the additives include, pharmaceutically acceptable ingredients and the like. There is no particular limitation for specific examples of the additives, but they include, for example, excipients, carriers (substrates), and the like. Examples of the excipients and the carriers include aqueous solvents such as water, physiological saline, and buffers; oils and fats such as soybean oil; vaseline; alcohols such as glycerol; sugars such as maltose, dextrose, and dextrin; sugar alcohols such as xylitol; phospholipids; liposomes; and the like. In addition to the above, examples of the additives include binders, disintegrants, surfactants, emulsifiers, antioxidants, lubricants, wetting agents, thickening agents, stabilizers, UV blockers, antiseptic agents, preservatives, vitamins, minerals, colorants, and the like. The additives may be selected as appropriate, for example, depending on the administration mode of the angiogenic agent according to the present invention.

There is no particular limitation for the dosage form of the angiogenic agent according the present invention, but it may be selected as appropriate, for example, depending on the administration mode. There is no particular limitation for the dosage form, but examples include those exemplified for the medicinal composition according to the present invention. They specifically include, for example, liquid, and the like.

The method of treating a living organism according to the present invention may be described as, for example, an angiogenesis method. In this case, the angiogenesis method according to the present invention comprises an administration step of administering the medicinal composition according to the present invention (i.e., the angiogenic agent as described above) to a living organism. The angiogenesis method according to the present invention is characterized by the use of the medicinal composition according to the present invention. There is no particular limitation for other steps and conditions. The administration step may be, for example, oral administration or parenteral administration. Examples include application to an affected area. The description about the angiogenic agent according to the present invention can be applied to the angiogenesis method according to the present invention.

### (5) Wound healing agent and method of treating wound

The medicinal composition according to the present invention can be used, for example, as a wound healing agent, and may also be referred to as a wound healing agent according to the present invention.

There is no particular limitation for the dosage of the wound healing agent according to the present invention, but the wound healing agent is preferably administered in a pharmaceutically effective amount. The pharmaceutically effective amount can be determined, for example, based on factors such as a wound site and the extent of a wound. There is no particular limitation for the content of the extracellular vesicles derived from equine mesenchymal stem cells in the wound healaing agent according to the present invention, but the content is preferably, for example, such that a pharmaceutically effective amount can be administered.

There is no particular limitation for the administration mode of the wound healing agent according to the present invention, but it may be, for example, oral or parenteral, and is preferably parenteral. A specific example is application to a wound.

Specific examples of conditions when applying the wound healing agent according to the present invention to an affected wound area (e.g., body surface such as skin) include, for example, the following:
The total daily amount of the extracellular vesicles: 1e+9 to 1e+10 particles
The application amount of the extracellular vesicles per unit area per application: 1e+8 to 1e+9 particles/cm²
The number of applications per day: 1 to 3
The duration of application: until a wound is completely recovered
The interval of applications: application daily, or every 2 to 3 days

The wound healing agent according to the present invention may include, for example, only extracellular vesicles derived from equine mesenchymal stem cells as an active ingredient, or may further include other ingredients, as described above. Further, the wound healing agent according to the present invention may include, for example, only the aforementioned active ingredient, or may include the aforementioned active ingredient and other additives, as described above. Examples of the additives include, pharmaceutically acceptable ingredients and the like. There is no particular limitation for specific examples of the additives, but they include, for example, excipients, carriers (substrates), and the like. Examples of the excipients and the carriers include aqueous solvents such as water, physiological saline, and buffers; oils and fats such as soybean oil; vaseline; alcohols such as glycerol; sugars such as maltose, dextrose, and dextrin; sugar alcohols such as xylitol; phospholipids; liposomes; and the like. In addition to the above, examples of the additives include binders, disintegrants, surfactants, emulsifiers, antioxidants, lubricants, wetting agents, thickening agents, stabilizers, UV blockers, antiseptic agents, preservatives, vitamins, minerals, colorants, and the like. The additives may be selected as appropriate, for example, depending on the administration mode of the wound healing agent according to the present invention.

There is no particular limitation for the dosage form of the wound healing agent according the present invention, but it may be selected as appropriate, for example, depending on the administration mode. Examples of the wound healing agent according to the present invention include topical agents (topical medications). In view of application, the dosage form of the wound healing agent according to the present invention may be, for example, liquid, emulsion, gel, sol, ointment, and the like.

The method of treating a living organism according to the present invention may be described as, for example, a method of treating a wound. In this case, the method of treating a wound according to the present invention comprises an administration step of administering the medicinal composition according to the present invention (i.e., the wound healing agent as described above) to a living organism. The wound healing method according to the present invention is characterized by the use of the medicinal composition according to the present invention. There is no particular limitation for other steps and conditions. The administration step may be, for example, oral administration or parenteral administration. Examples include application to an affected area. The description about the wound healing agent according to the present invention can be applied to the method of treating a wound according to the present invention.

As described above, the medicinal composition according to the present invention, which has functions such as tissue repair, angiogenesis, and anti-inflammation, and can promote, for example, wound healing, may also be referred to as a wound healing agent. The method of treating a living organism according to the present invention may be described as, for example, a method of healing a wound.

### (6) Applications

The applications of the present invention illustrated below can take advantage of the descriptions above.

The present invention is the use of extracellular vesicles derived from equine mesenchymal stem cells for use in tissue repair. Further, the present invention is the use of extracellular vesicles derived from equine mesenchymal stem cells for use in manufacture of a tissue repair agent.

The present invention is the use of extracellular vesicles derived from equine mesenchymal stem cells for use in the treatment of an inflammation. Further, the present invention is the use of extracellular vesicles derived from equine mesenchymal stem cells for use in manufacture of an anti-inflammatory agent.

The present invention is the use of extracellular vesicles derived from equine mesenchymal stem cells for use in angiogenesis. Further, the present invention is the use of extracellular vesicles derived from equine mesenchymal stem cells for use in manufacture of an angiogenic agent.

The present invention is the use of extracellular vesicles derived from equine mesenchymal stem cells for use in the healing of a wound. Further, the present invention is the use of extracellular vesicles derived from equine mesenchymal stem cells for use in manufacture of a wound healing agent.

The present invention will be described in detail below with reference to Examples and the like, but the present invention shall not be limited to these.

### EXAMPLES

### [Example 1]

Extracellular vesicles derived from equine mesenchymal stem cells were prepared by the following method, and analyzed.

### (1) Preparation method

Equine mesenchymal stem cells (EqMSC-ad) isolated from equine adipose tissues were used. The cells were purchased from ScienCells Research Laboratories (Product number: H7510, Lot No. 1396). As a cell proliferation medium, used was a basal medium for mesenchymal stem cells (Product Name: Mesenchymal Stem Cell Medium, Product number: 7501, ScienCells Research Laboratories)including a mesenchymal stem cell growth supplement (Product name: MSCGS, Product number: 7552, ScienCells Research Laboratories), an antibiotic (Product name; P/S Solution, Product number: 0503, ScienCells Research Laboratories), and 10% horse serum (Product name: Horse Serum, donor, Product number: S0900, BWT).

The basal medium in an amount of 10 mL was added to each of 7 culture dishes having a diameter of 10 cm. The equine mesenchymal stem cells with a passage number of 6 were seeded to the basal medium at 2e+5 cells/mL, and cultured at 37°C for one day. After confirming that the cells had adhered to the culture dish, the culture supernatant was removed. The cells were then washed with 10 mL of PBS (-) including no Ca²⁺ and Mg²⁺. To each of the culture dishes, 10 mL of extracellular vesicle recovery medium was added, and cultured at 37°C for 48 hours. As the extracellular vesicle recovery medium, used was a basal medium for mesenchymal stem cells which was free of phenol red and free of antimicrobial agents (Product name: MSCM-prf with MSCGS, ScienCells Research Laboratories).

The cultures from the 7 culture dishes were combined and centrifuged (2000×g, 10 minutes, room temperature) to collect the culture supernatant. This culture supernatant was passed through a filter tool with a pore size of 0.22 µm (Product name: STERICUP^{®}, Merck Millipore), and the filtrate was collected. The filtrate was dispensed to 6 centrifuge tubes (11 mL per tube), and ultracentrifuged (35,000 rpm, 70 minutes, 4°C) in an ultracentrifuge (Beckman Coulter). The supernatant was removed from each of the tubes, and 11 mL of the PBS (-) was added to each of the tubes to suspend the pellets. Again, these 6 tubes were ultracentrifuged at the same conditions in the ultracentrifuge. The supernatant was then removed from each of the tubes, and the pellet was collected from each of the tubes (500 µL in total). These pellets were used as a sample of extracellular vesicles derived from equine mesenchymal stem cells (hereinafter referred to as an "equine MSC-derived EVs sample") and used below.

### (2) Particle size distribution

The equine MSC-derived EVs sample was analyzed using a nanoparticle tracking system (Product name: NanoSight SN300, Quantum Design Japan) to determine the particle size distribution of the extracellular vesicles included in the equine MSC-derived EVs sample under the following conditions:
Measurement time: 60 seconds
Number of repetitions: 3 times
Detection threshold: 5
Camera type: sCMOS
Laser type: Blue405
Camera level: 13
Syringe pump speed: 40

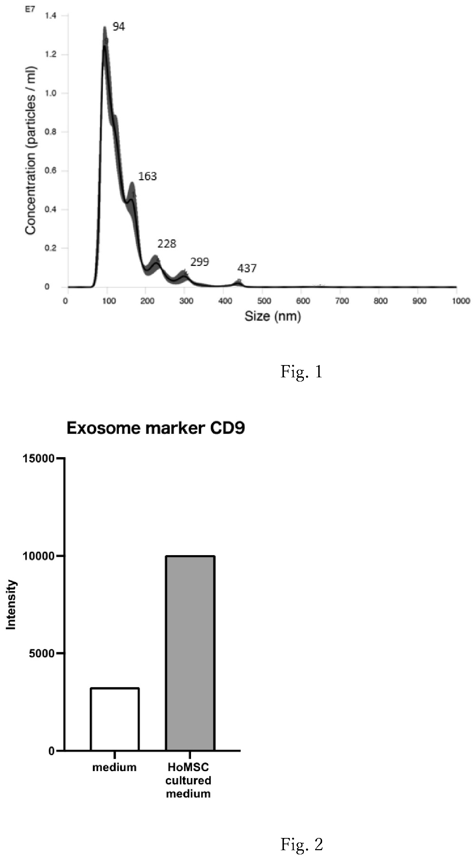

The result for the particle size distribution of the equine MSC-derived EVs sample is shown in FIG. 1 and the table below. Further, the electron microscopy of the equine MSC-derived EVs sample showed the presence of spherical particles having a particle size as shown in Table 1.

**Table 1**

| | Extracellular vesicle sample |
|---|---|
| Mean | 138.4 ± 1.7 nm |
| Mode | 93.2 ± 1.0 nm |
| Conc. (particles/mL) | 1.698e+10 |

### (3) Identification of extracellular vesicles

The culture supernatant obtained as described above was analyzed to detect a tetraspanin protein CD9 which can serve as a marker for exosomes. Detection of the exosome marker CD9 was performed using the ExoScreen method (Article Title: "Ultra-sensitive liquid biopsy of circulating extracellular vesicles using ExoScreen," Nat Commun, 2014, DOI: 10.1038/ncomms4591).

Biotin-labeled CD9 antibody and acceptor bead-labeled CD9 antibody were diluted with a universal buffer (including Product name: x1 universal buffer (Perkin Elmer) and 1 mg/ml dextran (Sigma)) to prepare a diluted liquid. To a well plate (Product name: 1/2 Area Plate 96, Perkin Elmer), 15 µL of the diluted liquid and 10 µL of the culture supernatant were added and allowed to react at 37°C for 1 hour. Then, 25 µL of donor beads (Perkin Elmer) diluted with the universal buffer was added to the plate, and again allowed to react at 37°C for 1 hour. The plate was analyzed to detect fluorescence at 520 to 620 nm indicative of the presence of CD9 using a detection instrument (Product name: Envision 2015 HTS, Perkin Elmer). In addition, DMEM free of fetal bovine serum was used as a control in place of the culture supernatant, and fluorescence intensity was detected in the same manner.

FIG. 2 shows the results of CD9 detection in the culture supernatant. In FIG. 2, the vertical axis represents a relative fluorescence intensity, and corresponds to CD9. As shown in FIG. 2, CD9 was detected in the culture supernatant, confirming the presence of the extracellular vesicles.

### [Example 2]

The equine MSC-derived EVs sample from Example 1 was analyzed for the efficacy of tissue repair.

Human fibroblasts were used to create a wound model. As a medium, used was of DMEM (GIBCO) including 10% fetal bovine serum and 1% antibiotic-antimycotic agent (Product name: Antibiotic-Antimycotic, GIBCO). The culture temperature was 37°C. Human fibroblasts were cultured in the medium, and then seeded to a 24-well plate (Product name: NUNC^{®} Cell-Culture Treated Multidishes, Product number: 142475, NUNC) at 2×10⁵ cells per well. Then the cells were further cultured. Once 100% confluence was reached, the medium was removed from the plate. Scars serving as a wound model were placed therein by scraping the surface of the cell sheet in the plate with the tip of a pipette, and the resulting cell clusters were washed with 500 µL of PBS (-).

The equine MSC-derived EVs sample was diluted in serum-free DMEM to give a concentration of 1e+9 particles/mL to prepare a diluted EVs sample. The diluted EVs sample was added to wells of the plate at 500 µL/well, and further cultured. The cell clusters in the wells were washed with PBS (-) 48 hours after the start of culture, and subjected to fixation with 300 µL of 4% PFA at room temperature for 15 minutes, removal of PFA, wash with PBS (-), and HE-staining. Then, the cell clusters after HE-staining (48 hours of culture) were imaged using a confocal microscope (Product name: MICA, Leica) (n=8). It is noted that the cell clusters immediately after the addition of the diluted EVs sample (0 hour of culture) were also subjected to HE staining and imaging (n=8) in the same manner. In addition, PBS (-) was added as a control in place of the extracellular vesicle sample, and the cells were cultured, HE-stained, and imaged in the same manner.

Then, a free software (Fiji) was used to calculate how much a wound area in the cell clusters (48 hours of culture) was reduced as compared to the wound area in the cell clusters (0 hour of culture). Specifically, the wound area in the cell clusters (0 hour of culture) was considered as 100%, and the percentage of a wound area after culturing was calculated as the extent of a wound (% area). A smaller % area, which is indicative of a smaller extent of a wound, means that the wound has reduced.

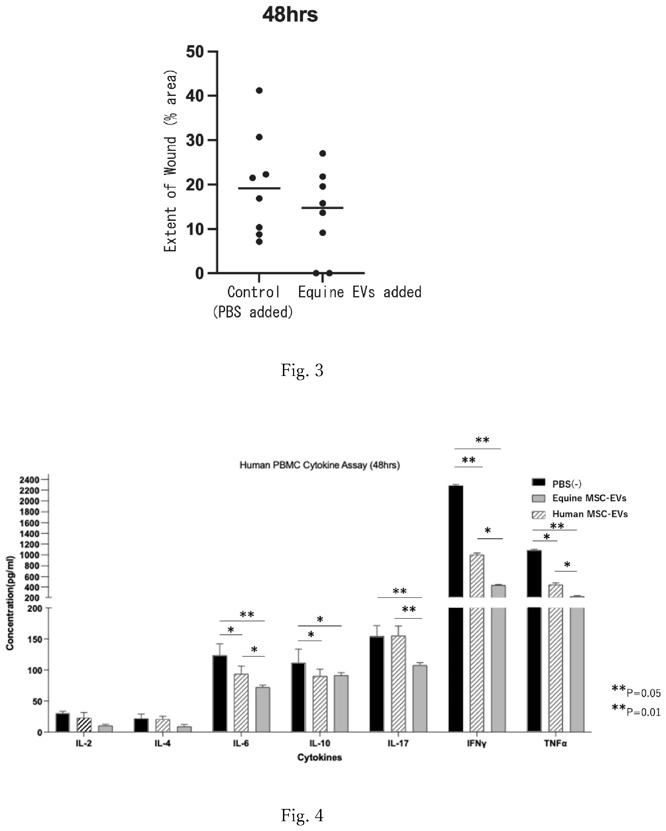

These results are shown in FIG. 3. FIG. 3 shows a graph of the extents of wounds. The vertical axis represents the extent of a wound, and indicates the percentage of the area of the remaining wound relative to the wound area prior to the start of culture. As seen in FIG. 3, the cell clusters treated with the equine MSC-derived EVs sample can show a reduced wound area as compared to the cell clusters treated with PBS (-). These results support that the equine MSC-derived EVs sample is capable of restoring a tissue structure per se to its original state, i.e., capable of achieving tissue repair by restoring cell functions at a wound site, and filling a wound with cells to promote regeneration and proliferation.

### [Example 3]

The equine MSC-derived EVs sample was analyzed for anti-inflammatory effects.

The equine MSC-derived EVs sample was used as a sample in Example 3. A human MSC-derived extracellular vesicle sample (hereinafter referred to as a human MSC-derived EVs sample) was used as a sample in Comparative Example 3. PBS (-) was used as a sample for a control.

The equine MSC-derived EVs sample was prepared as described below. Specifically, the same equine-derived mesenchymal stem cells as in Example 1 were cultured, and a culture with a number of passage (passage number) of 7 was subjected to centrifugation (2000×g, 10 minutes, room temperature) to collect a culture supernatant. That culture supernatant was concentrated by tangential flow filtration (TFF; the molecular weight cut-off of the membrane used was 300 kDa), and the resulting concentrate was further ultracentrifuged (35,000 rpm, 70 minutes, 4°C). The pellet was collected, and suspended in D-PBS (-) (Nissui Pharmaceutical Co., Ltd.; Cat. No. 05913), which was used as an equine MSC-derived EVs sample. It is noted that unless otherwise specified, the medium and the culture conditions as in Example 1 were used.

The equine MSC-derived EVs sample was analyzed for the particle size distribution of the extracellular vesicles as in Example 1. The results are shown in Table 2 below.

**Table 2**

| | Extracellular vesicle sample |
|---|---|
| Mean | 112.0 ± 1.4 nm |
| Mode | 85.2 ± 6.2 nm |
| Conc. (particles/mL) | 8.58e+08 ± 3.05e+06 |

The human MSC-derived EVs sample was prepared in the same manner as the equine MSC-derived EVs sample except that a culture supernatant from human adipose-derived mesenchymal stem cells (LONZA; Cat. No. PT-5006; Lot No. 647217) with a passage number of 5 was used.

Human peripheral blood mononuclear cells (PBMCs) were seeded to a 96-well plate at 1e+5 cells per well, which was then cultured. As a medium, used was RPMI 1640 medium (available from Thermo Fisher) supplemented with 10% fetal bovine serum and 2 mM (mmol/L) L-glutamine. Concanavalin A (Con-A) was added to the wells 24 hours after the start of culture to stimulate the PMBCs, thereby inducing cytokine storm (inflammation). Concanavalin A was added so as to give a final concentration of 5 µg/mL per well.

The PMBCs with induced inflammation were seeded to a 96-well plate at 1e+5 cells per well 24 hours after the addition of concanavalin A. The samples were each added to a fresh medium in the wells, and then cultured. The samples for this Example and this Comparative Example were added so that 1,000 particles of extracellular vesicles were present per PMBC cell, and 100 µL per well of physiological saline was added as a control. Then, the supernatants were collected from the cultures 48 hours after the addition of the samples, and cytokines (IL-2, IL-5, IL-10, IL-17, IFN-γ, TNF-α) in the supernatants were quantified using a commercially available ELISA kit.

These results are shown in FIG. 4. FIG. 4 shows a graph of the concentrations of the cytokines in the supernatants. As shown in FIG. 4, the system (n=3) to which the equine MSC-derived EVs sample (Equine MSC-EVs) from this Example was added showed reduced levels of all of the cytokines as compared to the control (n=3) and even as compared to the system (n=3) to which the human MSC-derived EVs sample (human MSC-EVs) from this Comparative Example was added.

### [Example 4]

The equine MSC-derived EVs sample was analyzed for angiogenic effects.

The equine MSC-derived EVs sample in Example 3 was used as the EVs sample in Example 4 while the human MSC-derived EVs sample in Comparative Example 3 was used as the EVs sample in Comparative Example 4.

EGM-2 supplemental factors (Lonza; Cat. No. CC-4176) was added to EBM-2 (Lonza; Cat. No. CC-3162) to prepare an EGM-2 medium. The supplemental factors were hEGF, VEGF, R3-IGF-1, ascorbic acid, hydrocortisone, hFGF-β, heparin, FBS, and gentamicin/amphotericin-B. Then, this EGM-2 medium was mixed with an EBM-2 medium (Lonza; Cat. No. CC-3162; without these supplemental factors) in a volume ratio of 1:1. Further, the EVs samples were added to this mixed medium to prepare EVs-containing media for Example 4 and Comparative Example 4, respectively. The EVs samples each were added so that the concentration of EVs per well (50 µL of medium) was 5.0e+7 particles/well. For the control, a control medium was used in which the same amount of PBS (-) as the EVs samples was added to the mixed medium.

Frozen human umbilical vein endothelial cells (HUVECs) were subjected to thawing, media exchange, and passaging, and then suspended in the corresponding medium. The suspended HUVECs were seeded to a 96-well plate (Thermo, Nunc Microwell 96F) coated with Matrigel (Corning, Cat. No. 356231) at 1.5e+4 cells/well, and cultured at 37°C for 16 hours. Triplicate wells were used for each of the media (n=3) Then, tubes formed by HUVECs after culturing were analyzed by an image analysis software (ImageJ) to measure their total lengths, and by phase-contrast microscopy to count the number of branching points. Then, the mean value for each system including the corresponding medium was compared among the control (EVs(-)), Example 4 (Equine MSC-EVs), and Comparative Example 4 (Human MSC-EVs).

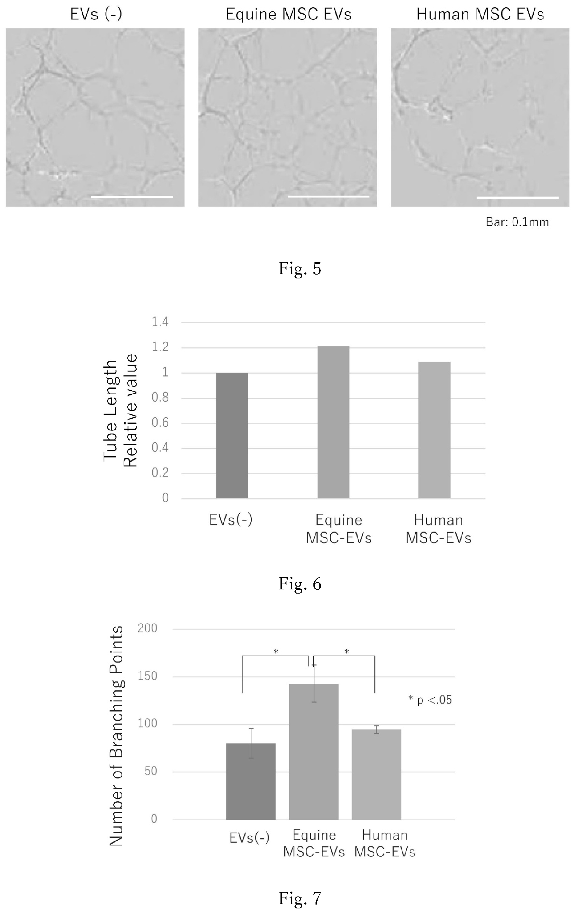

These results are shown in FIGs. 5, 6, and 7. FIG. 5 shows phase-contrast micrographs of HUVEC tubes. FIG. 6 shows the relative values of the lengths of HUVEC tubes. Specifically, these are relative values relative to a value of 1 for the control. FIG. 7 shows a graph of the number of branch points in the HUVEC tubes. As shown in these figures, the tube lengths when the equine MSC-derived EVs sample was used were found to be longer as compared to both the systems of the control and the human MSC-derived EVs sample. Further, the number of the branching points when the equine MSC-derived EVs sample was used was found to be significantly larger as compared to both the systems of the control and the human MSC-derived EVs sample. These demonstrate that the equine MSC-derived EVs can promote angiogenesis.

As described above, it was confirmed that the equine MSC-derived EVs according the present invention can exhibit a function of tissue repair as demonstrated in Example 2, a function of reducing inflammation as demonstrated in Example 2, and a function of promoting angiogenesis as demonstrated in Example 3. The equine MSC-derived EVs can be used for wound healing because these functions enable wound healing.

### [Example 5]

Extracellular vesicles derived from equine mesenchymal stem cells were prepared by the following method and analyzed for cell proliferation capability.

### (1) Preparation method

Equine mesenchymal stem cells (EqMSC-UC) isolated from equine umbilical cord (provided from Northern Farm) were used. Unless otherwise indicated, the culture method and the method of collecting extracellular vesicles were as in those described in Example 1.

The basal medium in an amount of 20 mL was added to each of 30 culture dishes having a diameter of 15 cm. The equine mesenchymal stem cells with the passage number of 6 were seeded to the basal medium at 3.5e+4 cells/mL, and cultured at 37°C for 2 days. After confirming that the cells had adhered to the culture dish, the culture supernatant was removed. The cells were then washed with 10 mL of PBS (-) including no Ca²⁺ and Mg²⁺. To each of the culture dishes, 20 mL of extracellular vesicle recovery medium was further added, and cultured at 37°C for 48 hours. As the extracellular vesicle recovery medium, used was DMEM (Product name D-MEM (High Glucose) without L-Glutamine and Phenol Red, FujiFilm Wako) free of phenol red and free of antimicrobial agents.

The cultures from the 7 culture dishes were combined, and centrifuged (2000×g, 10 minutes, room temperature) to collect the culture supernatant. The culture supernatant was passed through a filter tool with a pore size of 0.22 µm (Product name: STERICUP^{®}, Merck Millipore), and the filtrate was collected. This filtrate was used as a sample of extracellular vesicles derived from equine mesenchymal stem cells (hereinafter referred to as an equine MSC-derived EVs sample (EqMSC-UC CM)). This sample was diluted 5-fold in PBS (-), and analyzed using a nanoparticle tracking system (Product name: NanoSight SN300, Quantum Design Japan) as in Example 1 to determine the particle size distribution of the extracellular vesicles (EVs).

The results for the particle size distribution of the extracellular vesicles (EVs) of the equine MSC-derived EVs sample is shown in the table below. Further, the electron microscopy of the equine MSC-derived EVs sample showed the presence of spherical particles having a particle size as shown in the table below.

**Table 3**

| | Extracellular vesicle sample |
|---|---|
| Mean | 131.3 ± 1.7 nm |
| Mode | 96.6 ± 2.8 nm |
| Conc. (particles/mL) | 3.28e+09 ± 2.55e+07 |

### (2) Analysis of proliferation

The equine MSC-derived EVs sample (EqMSC-UC CM) described in (1) above was diluted with the phenol red-free DMEM as described above, and diluted samples at multiple dilution factors were prepared and used.

The following 3 types of cells were used as target cells for evaluation of proliferation:
Dermal Fibroblasts: Product number: CC-2059, from Lonza
IHFPDCs (dermal papilla cells): Product number: T0501, from abm
NHEKs (skin-derived keratinocytes): Product number: 00192907, from Lonza

These types of target cells were each seeded separately to a 96-well plate at 1000 cells/50 µL/well, and cultured overnight. The following media were used, respectively:
(Dermal Fibroblast)
   DMEM (Product name: D-MEM (High Glucose) (Product name: D-MEM (High Glucose) with L-glutamine, phenol red, and sodium pyruvate, FujiFilm Wako) including 10% fetal bovine serum and 1% antibiotic-antimycotic agent (Product name: Antibiotic-Antimycotic, GIBCO)
(IHFPDC)
   PriGrow III (Product number: TM003, abm) including 10% fetal bovine serum and 1% antibiotic-antimycotic agent
(NHEK)
   KGM Gold Keratinocyte Medium (Product number: 00192060, Lonza)

In this Example, a diluted sample of the EqMSC-UC CM was added at 50 µL/well, and the target cells were cultured. For each of the cell types, the concentration of the equine MSC-derived EVs per well (particles/well) was 2.5e+6, 4.10e+7, 8.20e+7, 1.6e+8, and 3.28e+8, respectively. In addition, for a control, 50 µL/well of phenol red-free DMEM only was added as a control sample instead of the diluted sample, and the target cells were cultured. A reagent (Product name: Cell Counting Kit-8, Dojindo Laboratories) was added at 10 µL/well 72 hours after adding the diluted sample or the control sample, and allowed to react at 37°C for 4 hours. After the reaction, the absorbance at 450 nm was measured for each well. Then, the relative value of absorbance for this Example was calculated and expressed as a cell viability (%) using the absorbance for the control which was considered as 100%.

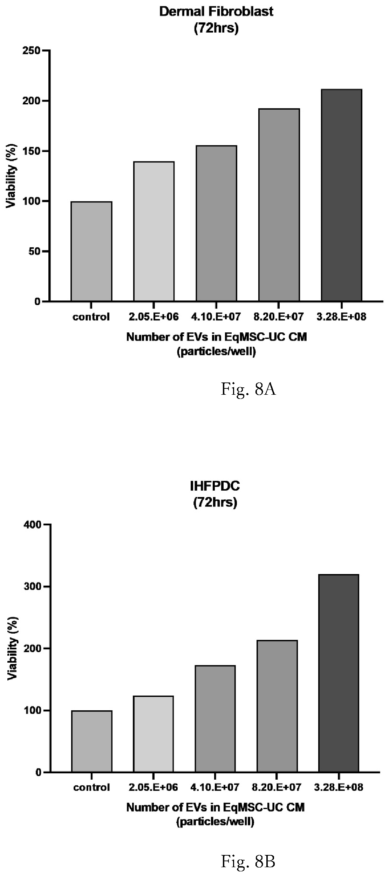

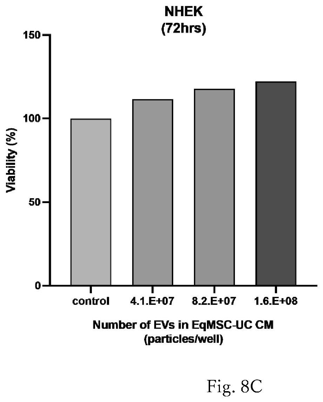

These results are shown in FIG. 8. FIG. 8A shows the cell viability of dermal fibroblasts, and FIG. 8B shows the cell viability of IHFPDCs (dermal papilla cells), and FIG. 8C shows the cell viability of NHEKs (skin-derived keratinocytes). FIGs. 8A, 8B, and 8C demonstrate that the addition of the equine MSC-derived EVs sample increased the cell viability in a concentration-dependent manner for all of the cell types, i.e., demonstrating that cell proliferation was promoted.

In wound healing, the proliferation of skin-derived fibroblasts is important, for example, in terms of tissue repair at a wound site such as the skin surface; and the proliferation of dermal papilla cells is important, for example, in terms of replenishment of dermal papilla cells lost due to trauma, ultraviolet radiation, and inflammation, activation of hair follicle cells, and activation of melanocytes; and the proliferation of skin-derived keratinocytes is important, for example, in terms of contribution to repairing an epidermal layer lost due to wounds. Therefore, as shown in FIG. 8, the addition of the equine MSC-derived EVs sample can promote the proliferation of each cell type, indicating that the equine MSC-derived EVs were effective for wound healing.

The present invention has been described with reference to the embodiments as described above, but the present invention shall not be limited to the above embodiments. As understood by those skilled in the art, various modifications may be made to the configurations and details of the present invention without departing from the scope of the present invention.

The present application claims the priority of Japanese Patent Application No. 2024-12210 filed on January 30, 2024, and Japanese Patent Application No. 2024-101298 filed on June 24, 2024, the disclosures of which are all incorporated herein by reference.

### [Industrial Applicability]

The medicinal composition according to the present invention can promote, for example, tissue repair, reduced inflammation, and angiogenesis in a living organism; and also enables wound healing by virtue of these functions.

## Claims

1. A medicinal composition **characterized by** comprising extracellular vesicles derived from equine mesenchymal stem cells.

2. The medicinal composition according to claim 1, wherein the medicinal composition is a tissue repair agent.

3. The medicinal composition according to claim 1 or 2, wherein the medicinal composition is an anti-inflammatory agent.

4. The medicinal composition according to any one of claims 1 to 3, wherein the medicinal composition is an angiogenic agent.

5. The medicinal composition according to any one of claims 1 to 4, wherein the medicinal composition is a wound healing agent.

6. A method of treating a living organism, the method **characterized by** comprising an administration step of administering the drug composition according to any one of claims 1 to 5 to the living organism.

7. The method of treating a living organism according to claim 6, wherein the administration step comprises administering the drug composition to the living organism as a tissue repair agent.

8. The method of treating a living organism according to claim 6, wherein the administration step comprises administering the drug composition to the living organism as an anti-inflammatory agent.

9. The method of treating a living organism according to claim 6, wherein the administration step comprises administering the drug composition to the living organism as an angiogenic agent.

10. The method of treating a living organism according to any one of claims 6 to 9, wherein the administration step comprises administering the drug composition to the living organism as a wound healing agent.

11. The method of treating a living organism according to any one of claims 6 to 10, wherein the living organism is a human or a non-human animal.

12. The method of treating a living organism according to claim 11, wherein the non-human animal is a horse.
